# EUROPEAN PATENT SPECIFICATION

(11) **EP 4 098 192 B1**
(45) Date of publication and mention of the grant of the patent: **23.04.2025**
(21) Application number: 21789098.7
(22) Date of filing: 16.03.2021
(51) Int. Cl.: A61B 5/145, A61B 5/1473, A61B 5/00, A61B 5/155

(54) **APPARATUS FOR MEASURING BIOMETRIC INFORMATION**
VORRICHTUNG ZUM MESSEN BIOMETRISCHER INFORMATIONEN
APPAREIL DE MESURE D'INFORMATIONS BIOMÉTRIQUES

(30) Priority: 13.04.2020 KR 20200044830
(43) Date of publication of application: 07.12.2022
(73) Proprietor: i-Sens, Inc., Seoul 06646 (KR)
(72) Inventor: KANG, Young Jea, Seoul 06646 (KR); LEE, David, Seoul 06646 (KR); JEONG, In Seok, Seoul 06646 (KR); KIM, Suk Joon, Seoul 06646 (KR)
(74) Representative: BCKIP Part mbB
(86) International application number: PCT/KR2021/003225
(87) International publication number: WO 2021/210792

(56) References cited:
- KR-A- 20130 022 542
- KR-A- 20140 094 912
- KR-A- 20150 001 225
- KR-A- 20150 057 388
- KR-A- 20160 132 750
- US-A- 4 009 721
- US-A- 5 267 569
- US-A- 5 704 365
- US-A1- 2008 249 385

## Description

### TECHNICAL FIELD

The present invention relates to a body-attachable-type apparatus for measuring biometric information, and more specifically relates to a body-attachable-type apparatus for measuring biometric information, the apparatus in which, by connecting an additional impedance to a sensor impedance, a level of noise generated during deformation or movement of the sensor can be reduced, thereby accurately measuring biometric information, and by removing noise affecting a response signal during deformation or movement of the sensor through the additional impedance, the biometric information can be accurately measured.

### BACKGROUND

Diabetes is a major cause of death and a cause of disability worldwide, and therefore, many people have health problems due to diabetes. Specially, diabetes is a serious disease that causes heart and kidney disease, blindness, nerve damage and high blood pressure. According to a long-term clinical study, the incidence of complications can be significantly reduced by appropriately managing blood glucose levels. Therefore, it is important to continuously manage diabetes, an important factor is self-monitoring of blood glucose levels.

In response to this demand, a self-diagnosis biometer in which a user can check a blood glucose level of the user by himself or herself has been widely distributed and used. A conventional blood glucose meter measures the blood glucose level of the user by putting the user's blood on a sensor strip, which is a test strip. Accordingly, the sensor strip with the blood is inserted into the blood glucose meter, and the blood glucose level measured through the sensor strip is displayed on the blood glucose meter.

At this time, the blood glucose meter receives an electrical signal generated by an electrochemical reaction between the collected blood and the reactant in the sensor strip, and measures the blood glucose level. Such a blood-collect-type blood glucose meter (finger prick method) helps diabetic patients to manage blood glucose, but it is difficult to accurately identify the blood glucose levels which are being frequently changed because it shows only the result at the time of the measurement.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

To overcome such a drawback, continuous glucose monitoring systems (CGMSs) inserted into the human body to measure a blood glucose level every few minutes have been developed, and therefore easily perform the management of diabetics and responses to an emergency situation.

The continuous blood glucose measurement system measures biometric information of blood glucose by collecting test substance such as blood of human body in a state that a part of a sensor is inserted in the human body. For this, a sensor transmitter measuring biometric information in a state of being attached to the human body and a communication terminal receiving the measured biometric information data from the sensor transmitter are comprised.

There is a problem in that, in a body-attachable-type biometric information measurement apparatus such as a sensor transmitter for continuous blood glucose measurement, because blood glucose information is measured in a state in which the sensor transmitter is attached to the body and a part of the sensor is inserted into the human body, whenever a person moves, a physical force, applied to the sensor or an area around the body part where the sensor is inserted, causes the sensor to be deformed or moved, as the sensor moves in this way, the noise may be included in the biometric information data measured by the sensor transmitter, and this affects the response signal generated by the sensor transmitter, thereby making difficult in accurately measuring the biometric information.

Document KR 2016 0 132 750 A discloses an electrode structure for a bio sensor comprising a motion electrode, which can invade an object to be examined, and a first and a second impedance electrodes, which can be in contact with the object to be examined. The motion electrode includes an enzyme for converting a first electric reaction into a second electric reaction, corresponding to a first electric stimulus, which is applied to the object to be examined. The impedance electrodes receive the first and second electric reactions from the object to be examined, and are mutually separated and arranged.

### DETAILED DESCRIPTION OF INVENTION

### Technical Problem

To solve the problem of the conventional apparatus of measuring biometric information, the purpose of the present invention is to provide an apparatus for measuring biometric information, the apparatus which can accurately measure the biometric information by reducing a level of noise generated during deformation or movement of the sensor in a body-attachable-type apparatus for measuring biometric information.

Another purpose of the present invention is to provide an apparatus for measuring biometric information, the apparatus in which, by removing a level of noise affecting a response signal during deformation or movement of the sensor through an additionally arranged electrical load as well as further removing noise from a response signal including high frequency components of the noise, the biometric information can be accurately measured.

Still another purpose of the present invention is to provide an apparatus for measuring biometric information, the apparatus in which, by arranging an electrical load at a position of a sensor or a measurement module or a position where the sensor and the measurement module are coupled according to various embodiment of the present invention, biometric information can be accurately measured.

Still another purpose of the present invention is to provide an apparatus for measuring biometric information, the apparatus, which measures a temperature of an electrical load, can accurately measure the biometric information by considering an additional impedance of a load unit which changes according to the temperature.

### Solution to Problem

To accomplish the purpose of the present invention, the present invention provides an apparatus for measuring biometric information in accordance with claim 1. According to the invention, the apparatus comprises: a sensor including a bioelectrode configured to be insertable into skin; a measurement module configured to apply a measurement power to the bioelectrode and measure the biometric information based on a response signal received from the bioelectrode; and a load unit configured to add an additional impedance to a sensor impedance of the sensor to relatively reduce a level of noise applied to the response signal by physical deformation of the sensor, wherein a level of the additional impedance is greater than a level of the sensor impedance.

Here, the bioelectrode comprises a working electrode and a reference electrode, and the load unit is connected in series with the working electrode, or is connected in series with the reference electrode, or is connected in series with the working electrode and the reference electrode, respectively.

In one embodiment, the load unit is arranged in series with the bioelectrode inside a housing of the measurement module.

In another embodiment, the load unit is formed at a body of the sensor and is formed as a tracer electrically connecting the bioelectrode and the measurement module.

In still another embodiment, the sensor and the measurement module are coupled separably to each other, and the load unit is formed as a connection terminal electrically connecting the sensor and the measurement module to each other when the sensor and the measurement module are coupled.

Here, the measurement module further comprise a low frequency band pass filter configured to filter a high frequency component of the noise from the response signal of which the level of the noise is reduced by the load unit.

Here, the load unit is a resistor of 10KΩ to 10GΩ.

In the present invention, the measurement module comprises a temperature sensor configured to measure a temperature of the load unit, and the biometric information is measured from the response signal considering the additional impedance of the load unit that is changed according to the temperature measured by the temperature sensor.

### Advantageous Effects of Invention

An apparatus for measuring biometric information according to the present invention has the following effects.

First, an apparatus for measuring biometric information according to the present invention, by connecting an additional impedance to a sensor impedance in a body-attachable-type apparatus for measuring biometric information, reduces a level of noise generated during deformation or movement of the sensor, thereby accurately measuring the biometric information.

Second, an apparatus for measuring biometric information can accurately measure the biometric information by removing a level of noise affecting a response signal during deformation or movement of the sensor through an additionally arranged electrical load as well as further removing noise from a response signal including high frequency components of the noise.

Third, an apparatus for measuring biometric information can accurately measure the biometric information according to various embodiments, by arranging an electrical load at a position of a sensor or a measurement module or a position where the sensor and the measurement module are coupled.

### BRIEF DESCRIPTION OF THE DRAWINGS

FIG. 1 is a schematic diagram for illustrating a body attachable type continuous biometric information measurement system according to an embodiment of the present disclosure.
FIG. 2 is a diagram for illustrating an applicator for attaching a biometric information measurement apparatus to a human body according to an embodiment of the present invention.
FIG. 3 is a diagram for illustrating an Randles equivalent circuit of a sensor.
FIG. 4 is a functional block diagram for illustrating a biometric information measurement apparatus that can reduce the size of noise generated by movement of a sensor according to an embodiment of the present invention.
FIG. 5 is a diagram for explaining an example of connecting a load in a biometric information measurement device according to the present invention.
FIG. 6 is a diagram for explaining an example of a bioelectrode measurement apparatus according to the present invention.
FIG. 7 is a diagram for explaining another example of a biometric information measurement apparatus according to the present invention.
FIG. 8 is a diagram for explaining another example of a biometric information measurement apparatus according to the present invention.
FIG. 9 is a diagram for explaining another example of a biometric information measurement apparatus according to the present invention.

### DESCRIPTION OF EMBODIMENTS OF DISCLOSURE

The technical terms used in the present description are only for the purpose of describing exemplary embodiments, and they are not intended to limit the present invention. Also, unless otherwise defined, all technical terms used herein should be construed as having the same meaning as commonly understood by those skilled in the art, and should not be interpreted as being excessively inclusive or excessively restrictive. In addition, when a technical term used herein is an erroneous technical term that does not accurately represent the idea of the present invention, it should be understood as replacing the term by a technical term which can be properly understood by those skilled in the art.

Further, singular expressions used in the present specification include plural expressions unless they have definitely opposite meanings. In the present application, it shall not be construed that terms, such as "including" or "comprising", various constituent elements or steps described in the specification need to be all essentially included, and it shall be construed that some constituent elements or steps among the various constituent elements or steps may be omitted, or additional constituent elements or steps may be further included.

Hereinafter, with reference to the enclosed drawings, a body attachable type biometric information measurement apparatus according to an embodiment of the present invention is described in detail.

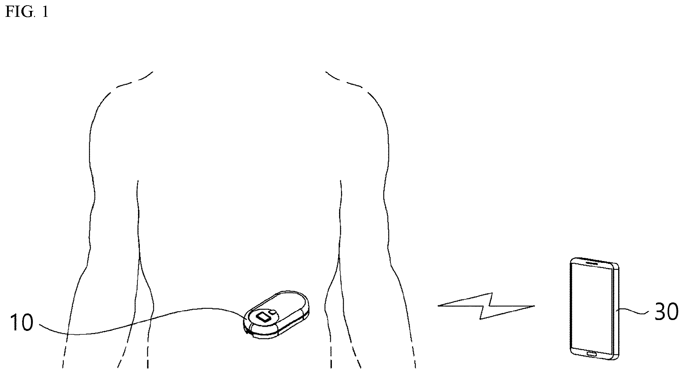

FIG. 1 is a schematic diagram for illustrating a body attachable type continuous biometric information measurement system according to an embodiment of the present disclosure.

Referring to FIG. 1, the continuous biometric information measurement system according to an embodiment of the present disclosure comprises a biometric information measurement apparatus (10) and a communication terminal (30).

The biometric information measurement apparatus (10) includes a sensor having a bioelectrode configured to be insertable into the body and a measuring module configured to measure biometric information based on a response signal received from the bioelectrode. When the biometric information measurement apparatus (10) is attached to the body, the bioelectrode of the sensor is inserted into the skin to generate an electrochemical reaction and generates a response signal, and the measurement module measures biometric information such as blood glucose and so on based on the response signal. The measurement module transmits the measured biometric information to the communication terminal in real time or periodically or when the communication terminal requests it.

Here, the sensor and the measurement module may be manufactured in an integral type, but the sensor and the measurement module may be manufactured to be separable from or couplable to each other. When the sensor and the measurement module are manufactured to be separable from or couplable to each other, the sensor and the measurement module have connection terminals that electrically contact each other, and a response signal generated by the sensor is provided to the measurement module through the connection terminals.

The communication terminal (30) is a terminal configured to receive biometric information from the biometric information measurement apparatus (10) and output or display the received biometric information to a user, and for example, the biometric information measurement apparatus (10) may be a portable terminal (such as smartphone, tablet PC, or notebook and so on) configured to communicate with the biometric information measurement apparatus (10). However, the communication terminal (30) is not limited thereto, and may be any type of a terminal which has a communication function and program or application can be installed to.

The biometric information measurement apparatus (10) transmits the biometric information in response to request of the communication terminal (30) or at predetermined times periodically, and for data communication between the biometric information measurement apparatus (10) and the communication terminal (30), the biometric information measurement apparatus (10) and the communication terminal (30) are communicationally connected to each other over a wire by an USB cable and so on or communicationally connected in an wireless communication means such as infrared communication, NFC communication, Bluetooth, etc.

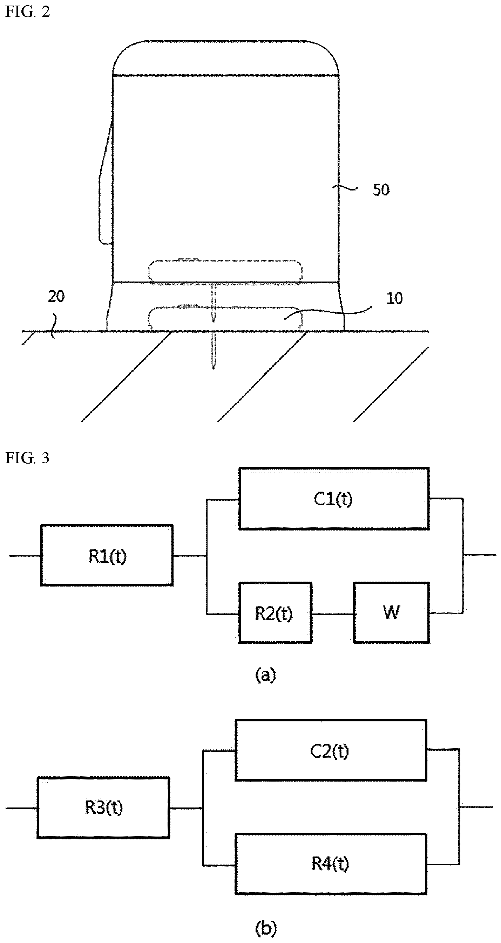

Here, the biometric information measurement apparatus is attached to a part of the human body by an applicator, and FIG. 2 is a diagram for illustrating an applicator for attaching a biometric information measurement apparatus to a human body according to an embodiment of the present invention.

An application (50) is now described by referring to FIG. 2, the biometric information measurement apparatus (10) is mounted in the applicator (50), and the applicator (50) can be operated so that the biometric information measurement apparatus (10) can be outwardly discharged to the outside of the applicator (50) by the manipulation of the user and then be attached to a specific portion of the human body of the user. The applicator (50) is formed to have a shape that one side of the applicator (50) is open, and the biometric information measurement apparatus (10) is installed to the applicator (50) through the open side of the applicator (50).

When the biometric information measurement apparatus (10) is attached to a part of the human body using the applicator (50), for inserting an end portion of the sensor included in the biometric information measurement apparatus (10) to skin, the applicator (50) comprises a needle (not shown) formed to cover the end portion of the sensor therein, a first elastic means (not shown) pushing the needle and the end portion of the sensor together towards the skin, and a second elastic means (not shown) configured to retract the needle only. The compressed state of the first elastic means (not shown) arranged to be compressed inside the applicator (50) by the configuration of the applicator (50) can be released, thereby inserting the needle and the end portion of the sensor simultaneously to the skin, and when the end portion of the sensor is inserted to the skin, the compressed state of the second elastic means (not shown) is released, thereby extracting the needle only. By the applicator (50), the user can safely and easily attach the biometric information measurement apparatus (10) to the skin.

An adhesive tape is provided at a surface of the biometric information measurement apparatus (10) contacting the human body so that the biometric information measurement apparatus (10) can be attached to the skin. Accordingly, if the applicator (50) is moved away from the skin of the human body, the biometric information measurement apparatus (10) is fixedly attached to the skin of the human body by the adhesive tape.

After that, if the power is supplied to the biometric information measurement apparatus (10), the biometric information measurement apparatus (10) is communicationally connected with the communication terminal (30), and the biometric information measurement apparatus (10) transmits the measured biometric information to the communication terminal (30).

FIG. 3 is a diagram for illustrating an Randles equivalent circuit of a sensor. As shown in FIG. 3(a), the Randles equivalent circuit is an electric circuit for modeling an electrochemical reaction occurring between a bioelectrode and an electrolyte, that is, an interface of a bodily fluid, and R1(t) is a resistance of the body fluid, C1(t) is a capacitance by an electric double layer, R2(t) is a charge transfer resistance, and W is a Warburg impedance.

If a film is positioned on the movement path of ions as shown in FIG. 3(b), in the Randles equivalent circuit, the sum of impedances such as resistance, capacitance and so on is a sensor impedance. In a body insertable type sensor, an electrode where an electrochemical reaction occurs is not directly exposed to the sensor, but a diffusion membrane controlling a diffusion rate of a target material and a biocompatible protective layer suppressing a biological immune response has a structure surrounding the sensor. These films can be added in series at a R1(t) position corresponding to a resistance of the body fluid in the Randles circuit. It is common to interpret these films as a parallel structure of a resistor and a capacitor. One of the many cases in which noise is generated by an external force applied to the sensor is changing components of the equivalent circuit shown in FIG. 3.

In particular, when the components corresponding to R1, R3, R4, and C2 of the equivalent circuit are changed instantaneously, it causes a change in the charging current of the sensor, and the response signal is changed instantaneously.

Because the biometric information measurement apparatus (10) is attached to the body and biometric information is measured in a state in which the bioelectrode of the sensor is inserted into the body, whenever the user moves, a physical force is applied to the sensor or the body part adjacent to where the sensor is inserted, thereby deforming or moving the sensor, and in this process, impedance components expressed with the equivalent circuit of the sensor are changed by an external force, noise may be included in the response signal generated by the sensor according to the change in the impedance component, and in this way, when the biometric information is measured from the response signal including the noise, the biometric information of the user cannot be accurately measured.

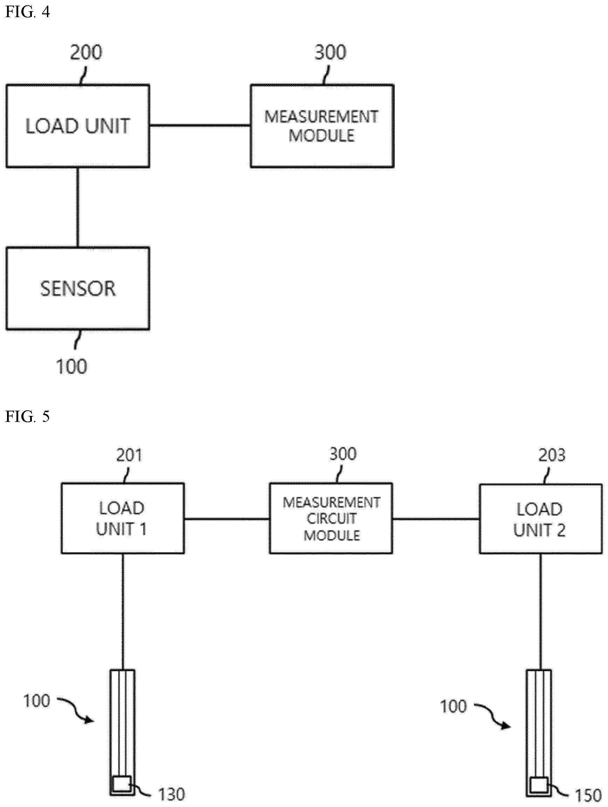

FIG. 4 is a functional block diagram for illustrating a biometric information measurement apparatus that can reduce the size of noise generated by movement of a sensor according to an embodiment of the present invention.

Describing more in detail with reference to FIG. 4, a bioelectrode is formed in the sensor (100), and the bioelectrode is inserted into the body, thereby causing an oxidation-reduction reaction and generating a response signal. A measurement module (300) provides a measurement power to the bioelectrode of the sensor (100) and measures biometric information based on the response signal received from the bioelectrode of the sensor (100). The measurement module (300) includes a communicator to communicate with the communication terminal, and the measurement module (300) transmits the measured biometric information to the communication terminal in real time, or transmits the measured biometric information to the communication terminal through the communicator when the communication terminal requests.

The biometric information measurement apparatus according to an embodiment of the present invention includes a load unit (200) configured to relatively suppress a noise level applied to the response signal generated by the sensor (100) when the sensor (100) is physically deformed by providing an additional impedance to a sensor impedance of the sensor (100). Here, the magnitude of the additional impedance of the load unit (200) is larger than the magnitude of the sensor impedance of the sensor (100). Preferably, it is characterized in that the load unit (200) is a resistor of 10KΩ to 10GΩ.

Like this, by connecting an additional impedance relatively larger than the sensor impedance to the sensor impedance in addition to the sensor impedance of the sensor (100) using the load unit (200), the effect of the change in the sensor impedance according to the physical change of the sensor (100) becomes relatively low in the overall impedance of the biometric information measurement apparatus, and due to this, even if the physical deformation occurs in the sensor (100), the amount of noise applied to the generated response signal can be reduced.

As shown in FIG. 5, a working electrode and a counter electrode (150) are formed at one end portion of the sensor, and a load unit 1 (201) is connected only to the working electrode and a separate load is not connected to the counter electrode (150), or a load part 2 (203) is connected only to the counter electrode (150) and a separate load is not connected to the working electrode , or the load unit 1 (201) and the load unit 2 (203) are connected to the working electrode and the counter electrode (150), respectively. In this way, even if the load is connected only to the working electrode , or the load is connected only to the counter electrode (150), or the loads are connected separately to the working electrode and the counter electrode 150, respectively, an additional impedance relatively larger than a sensor impedance is connected to the sensor impedance in addition to an sensor impedance of the sensor, and, due to this, the effect of the change in the sensor impedance changing according to the physical change of the sensor (100) becomes relatively low in the overall impedance of the biometric information measurement apparatus.

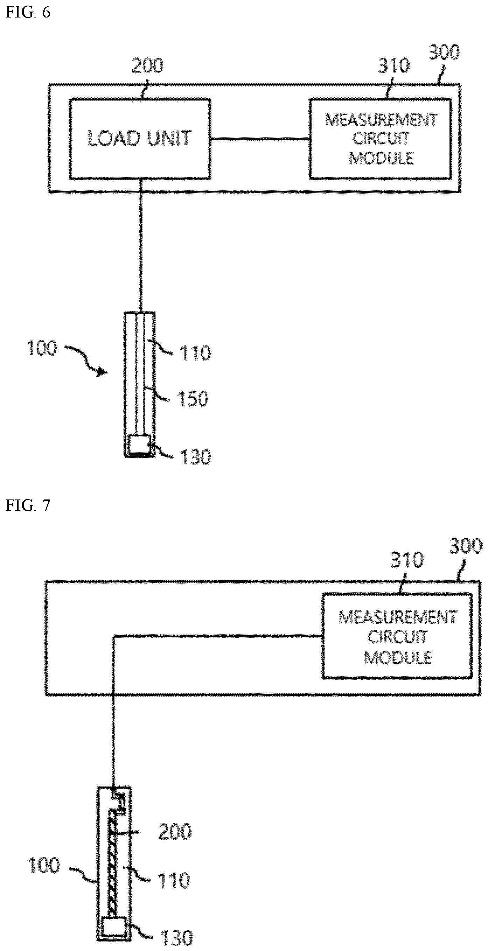

FIG. 6 is a diagram for explaining an example of a bioelectrode measurement apparatus according to the present invention.

Describing in more detail with respect to FIG. 6, a load unit (200) may be manufactured by being arranged inside a housing of a measurement module (300).

The sensor (100) includes a flexible base substrate (110), a bioelectrode (130) disposed at one end portion of the base substrate (110), and a conductive tracer (150) formed on an upper surface of the base substrate (110). When the sensor is attached to the body, the bioelectrode (130) disposed at one end portion of the base substrate (110) is inserted into the skin, thereby occurring electrochemical reaction inside the body, and generates a response signal by the electrochemical reaction, and the generated response signal is transmitted along the conductive tracer (150) to a measurement circuit unit (310) of the measurement unit (300). Here, the measurement circuit unit (310) provides a measurement power to the bioelectrode (130) and measures biometric information of the user by receiving a response signal according to an electrochemical reaction from the bioelectrode according to the measurement power, and the measurement circuit unit (310) stores the measured biometric information in an internal memory or transmits the measured biometric information to the communication terminal.

A load unit (200) is connected to the sensor (100) to provide additional impedance to the sensor impedance of the sensor (100), and therefore, this can contribute to reduce a level of noise generated when a shape of the sensor is deformed or the sensor moves according to a physical load applied to the sensor when a response signal is continuously generated by attaching the sensor to the body. That is, the magnitude of the total impedance in a view of the measurement circuit unit (310) is the sum of the sensor impedance of the sensor (100) and the additional impedance of the load unit

(200), and the magnitude of the additional impedance is relatively greater than the magnitude of the sensor impedance. Therefore, even if the sensor impedance is changed due to deformation or movement in or of the sensor, the effect or contribution of the change in the sensor impedance with respect to the overall impedance is small, and accordingly, the amount of noise generated in the response signal generated by the sensor can be reduced.

FIG. 7 is a diagram for explaining another example of a biometric information measurement apparatus according to the present invention.

Describing in detail with reference to FIG. 7, a load unit (200) may be manufactured as a conductive tracer of the sensor (100).

A sensor (100) includes a flexible base substrate (110), a bioelectrode (130) disposed at one end portion of the base substrate (110), and a conductive tracer formed on the upper surface of the base substrate (110), and in another example of a biometric information measurement apparatus according to the present invention, a conductive trace may be manufactured as the load unit (200) instead of a separate load unit. When the sensor is attached to the body, the bioelectrode (130) disposed on one end portion of the base substrate 110 is inserted into the skin, this causes an electrochemical reaction inside the body, thereby generating a response signal, and the generated response signal is transmitted along the conductive tracer to the measurement circuit unit (310) of the measurement unit (300).

Typically, a conductive tracer is formed to have an impedance as small as possible, but in another example of a biometric information measurement apparatus according to the present invention, a conductive tracer is manufactured to have a relatively high impedance in order to operate the conductive tracer as the load unit (200). Preferably, in order to have a high impedance so that the conductive tracer operates as a load part, the conductive tracer may be manufactured to have a high impedance by varying a material, shape, width, and length of the conductive tracer and so on.

The load unit (200) provides an additional impedance to the sensor impedance of the sensor (100) in order to contribute to reduce a level of noise generated when a shape of the sensor is deformed or the sensor moves according to a physical load applied to the sensor when a response signal is continuously generated by attaching the sensor to the body. That is, the magnitude of the total impedance in a view of the measurement circuit unit (310) is the sum of the sensor impedance of the sensor (100) and the additional impedance of the load unit (200), and the magnitude of the additional impedance is relatively greater than the magnitude of the sensor impedance. Therefore, even if the sensor impedance is changed due to deformation or movement in or of the sensor, the effect or contribution of the change in the sensor impedance with respect to the overall impedance is small, and accordingly, the amount of noise generated in the response signal generated by the sensor can be reduced.

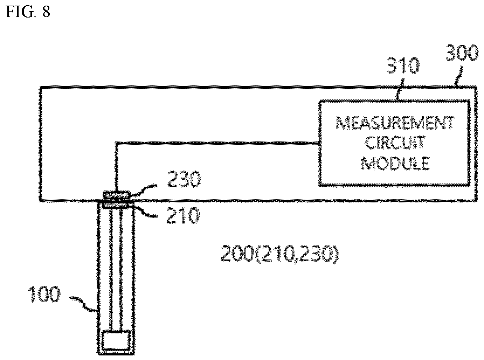

FIG. 8 is a diagram for explaining another example of a biometric information measurement apparatus according to the present invention.

Describing in detail with reference to FIG. 8, the sensor (100) and the measurement unit (300) are manufactured independently and are manufactured to be separable from or couplable to each other, and connection terminals (210, 230) for electrically connecting the sensor (100) and the measurement unit (300) when being coupled to each other are formed at the sensor (100) and the measurement unit (300). That is, the first connection terminal (210) is formed at one end of the sensor (100) couplable with the measurement unit (300), and the second connection terminal (230) for electrically connecting the measurement unit (300) with the sensor (100) is formed at the measurement unit (300). A response signal generated from the bioelectrode of the sensor (100) is transmitted to the first connection terminal (210) through the conductive tracer, and the second connection terminal (230) electrically connected to the first connection terminal (210) receives the response signal and transmits it to the measurement circuit unit (310).

Typically, a connection terminal uses a conductive elastic body with low impedance, but in another example of a biometric information measurement apparatus according to the present invention, connection terminals are manufactured to have a relatively high impedance in order to operate connection terminals (210, 230) as the load unit (200). Preferably, in order to have a high impedance so that the connection terminals (210, 230) can operate as a load part, the connection terminals (210, 230) may be manufactured to have a high impedance by varying a material, shape, width, and length of the connection terminals (210, 230) and so on.

The load unit (200) provides an additional impedance to the sensor impedance of the sensor (100) in order to contribute to reduce a level of noise generated when a shape of the sensor is deformed or the sensor moves according to a physical load applied to the sensor when a response signal is continuously generated by attaching the sensor to the body. That is, the magnitude of the total impedance in a view of the measurement circuit unit (310) is the sum of the sensor impedance of the sensor (100) and the additional impedance of the load unit (200), and the magnitude of the additional impedance is relatively greater than the magnitude of the sensor impedance. Therefore, even if the sensor impedance is changed due to deformation or movement in or of the sensor, the effect or contribution of the change in the sensor impedance with respect to the overall impedance is small, and accordingly, the amount of noise generated in the response signal generated by the sensor can be reduced.

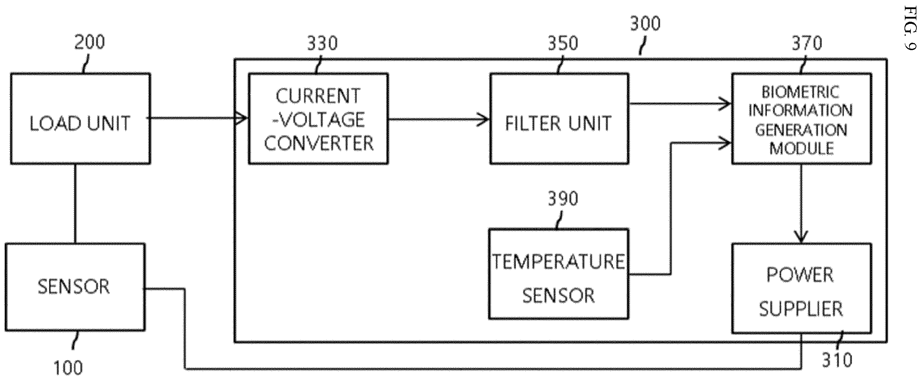

FIG. 9 is a diagram for explaining another example of a biometric information measurement apparatus according to the present invention.

Describing in detail with reference to FIG. 9, the measurement unit (300) includes a power supplier (310), a current-voltage converter (330), a biometric information generation module (370), and a temperature sensor (390).

The power supplier (310) applies a measurement power to the sensor, and the current-voltage converter (330) receives an analog current of a response signal from the sensor and converts it into a voltage of a measurement signal. Here, the current-voltage converter (330) may be OPAMP. The biometric information generation module (370) converts the analog measurement signal to digital to generate biometric information. The level of an electrical load of the load unit (200), that is, the level of an additional impedance, is changed according to the temperature of the load unit (200), and the temperature sensor (390) measures a temperature of the load unit itself, and provides information on the measured temperature to the biometric information generation module (370). The biometric information generation module (370) determines the level of the additional impedance in consideration of the temperature of the load unit (200) based on the measured temperature, and the biometric information can be accurately measured from the response signal based on the determined level of the additional impedance.

The measurement circuit unit described above with reference to FIGS. 5 to 8 includes a power supplier (310), a current-voltage converter (330), a biometric information generation module (370) and a temperature sensor (390), and can operate in the same manner.

Preferably, another example of a biometric information measurement apparatus according to the present invention further includes a filter unit (350), and the filter unit (350) filters a measurement signal converted into a voltage with a low-frequency band-pass filter to remove a high-frequency component of noise from the measurement signal.

That is, a biometric information measurement apparatus according to the present apparatus described with reference to FIG. 9 further includes a filter unit (350), by adding an additional impedance to a sensor impedance through the load unit (200), even if the sensor impedance is changed, the contribution of the change in the sensor impedance becomes small, thereby reducing the level of the noise in a current of a response signal applied to the current-voltage converter (330), and additionally, by removing high-frequency components of the noise from the measurement signal converted through the filter unit (350), the biometric information can be measured more accurately. Furthermore, OPAMP is used as the current-voltage converter (330), the current of the response signal of which the level of the noise is reduced through the load unit (200) is amplified by the current-voltage converter (330) to generate a voltage of the measurement signal, therefore through the combination of the load unit (200) and the filter unit (350), the amplification rate of the current-voltage converter (330) can be increased to reduce the measurement range, and it is possible to accurately measure the biometric information by being less affected by noise than simply using the filter unit (350) alone.

The present invention has been described with reference to embodiments shown in the drawings, but this is only exemplary, and it will be understood by those skilled in the art that various modifications are possible therefrom. Accordingly, the protection scope of the present invention is determined by the appended claims.

## Claims

1. An apparatus (10) for measuring biometric information, the apparatus (10) comprising:
a sensor (100) including a bioelectrode (130) configured to be insertable into skin (20);
a measurement module (300) configured to apply a measurement power to the bioelectrode (130) and measure the biometric information based on a response signal received from the bioelectrode (130); and
a load unit (200) configured to add an additional impedance to a sensor impedance of the sensor (100) to relatively reduce a level of noise applied to the response signal by physical deformation of the sensor (100),
wherein a level of the additional impedance is greater than a level of the sensor impedance, and
**characterized in that** the measurement module (300) comprises a temperature sensor (390) configured to measure a temperature of the load unit (200), and
**in that** the apparatus is configured to measure the biometric information from the response signal considering the additional impedance of the load unit (200) that is changed according to the temperature measured by the temperature sensor (390).

2. The apparatus (10) for measuring biometric information according to claim 1, wherein:
the bioelectrode (130) comprises a working electrode and a reference electrode, and
the load unit (200) is connected in series with the working electrode, or is connected in series with the reference electrode, or is connected in series with the working electrode and the reference electrode, respectively.

3. The apparatus (10) for measuring biometric information according to claim 1 or 2, wherein the load unit (200) is arranged in series with the bioelectrode (130) inside a housing of the measurement module (300).

4. The apparatus (10) for measuring biometric information according to any one of claims 1 to 3, wherein the load unit (200) is formed at a body of the sensor (100) and is formed as a tracer electrically connecting the bioelectrode (130) and the measurement module (300).

5. The apparatus (10) for measuring biometric information according to any one of claims 1 to 4, wherein:
the sensor (100) and the measurement module (300) are coupled separably to each other, and
the load unit (200) is formed as a connection terminal (210, 230) electrically connecting the sensor (100) and the measurement module (300) to each other when the sensor (100) and the measurement module (300) are coupled.

6. The apparatus (10) for measuring biometric information according to any one of claims 1 to 5, wherein the measurement module (300) further comprise a low frequency band pass filter configured to filter a high frequency component of the noise from the response signal of which the level of the noise is reduced by the load unit (200).

7. The apparatus (10) for measuring biometric information according to any one of claims 1 to 6, wherein the load unit (200) is a resistor of 10KΩ to 10GΩ.

## Patentansprüche

1. Vorrichtung (10) zum Messen biometrischer Informationen, wobei die Vorrichtung (10) umfasst:
einen Sensor (100), der eine Bioelektrode (130) beinhaltet, die so ausgebildet ist, dass sie in die Haut (20) einführbar ist;
ein Messmodul (300), das dazu ausgebildet ist, einen Messstrom an die Bioelektrode (130) anzulegen und die biometrischen Informationen basierend auf einem von der Bioelektrode (130) empfangenen Antwortsignal zu messen; und
eine Lasteinheit (200), die dazu ausgebildet ist, eine zusätzliche Impedanz zu einer Sensorimpedanz des Sensors (100) hinzuzufügen, um ein Rauschniveau, das auf das Antwortsignal durch physische Verformung des Sensors (100) angewandt wird, relativ zu reduzieren,
wobei ein Niveau der zusätzlichen Impedanz höher ist als ein Niveau der Sensorimpedanz, und
**dadurch gekennzeichnet, dass** das Messmodul (300) einen Temperatursensor (390) umfasst, der dazu ausgebildet ist, eine Temperatur der Lasteinheit (200) zu messen, und
die Vorrichtung dazu ausgebildet ist, die biometrischen Informationen aus dem Antwortsignal unter Berücksichtigung der zusätzlichen Impedanz der Lasteinheit (200) zu messen, die entsprechend der durch den Temperatursensor (390) gemessenen Temperatur verändert ist.

2. Vorrichtung (10) zum Messen biometrischer Informationen nach Anspruch 1, wobei:
die Bioelektrode (130) eine Arbeitselektrode und eine Referenzelektrode umfasst, und
die Lasteinheit (200) in Reihe mit der Arbeitselektrode verbunden ist oder in Reihe mit der Referenzelektrode verbunden ist oder jeweils in Reihe mit der Arbeitselektrode und der Referenzelektrode verbunden ist.

3. Vorrichtung (10) zum Messen biometrischer Informationen nach Anspruch 1 oder 2, wobei die Lasteinheit (200) in Reihe mit der Bioelektrode (130) innerhalb eines Gehäuses des Messmoduls (300) angeordnet ist.

4. Vorrichtung (10) zum Messen biometrischer Informationen nach einem der Ansprüche 1 bis 3, wobei die Lasteinheit (200) an einem Körper des Sensors (100) ausgebildet ist und als Tracer ausgebildet ist, der die Bioelektrode (130) und das Messmodul (300) elektrisch verbindet.

5. Vorrichtung (10) zum Messen biometrischer Informationen nach einem der Ansprüche 1 bis 4, wobei:
der Sensor (100) und das Messmodul (300) trennbar miteinander gekoppelt sind, und
die Lasteinheit (200) als ein Verbindungsanschluss (210, 230) ausgebildet ist, der den Sensor (100) und das Messmodul (300) elektrisch miteinander verbindet, wenn der Sensor (100) und das Messmodul (300) gekoppelt sind.

6. Vorrichtung (10) zum Messen biometrischer Informationen nach einem der Ansprüche 1 bis 5, wobei das Messmodul (300) weiterhin einen Niederfrequenz-Bandpassfilter umfasst, der dazu ausgebildet ist, eine Hochfrequenzkomponente des Rauschens aus dem Antwortsignal zu filtern, dessen Rauschniveau durch die Lasteinheit (200) reduziert ist.

7. Vorrichtung (10) zum Messen biometrischer Informationen nach einem der Ansprüche 1 bis 6, wobei die Lasteinheit (200) ein Widerstand von 10 kΩ bis 10 GΩ ist.

## Revendications

1. Appareil (10) de mesure d'informations biométriques, ledit appareil (10) comprenant:
un capteur (100) comprenant une bioélectrode (130) prévue pour être insérable sous la peau (20) ;
un module de mesure (300) prévu pour appliquer une puissance de mesure à la bioélectrode (130) et mesurer les informations biométriques sur la base d'un signal de réponse reçu de la bioélectrode (130); et
une unité de charge (200) prévue pour ajouter une impédance additionnelle à une impédance du capteur (100) afin de réduire relativement un niveau de bruit appliqué au signal de réponse par déformation physique du capteur (100),
où le niveau d'impédance additionnelle est supérieur au niveau d'impédance du capteur, et
**caractérisé en ce que** le module de mesure (300) comprend un capteur de température (390) prévu pour mesurer une température de l'unité de charge (200), et
**en ce que** l'appareil est prévu pour mesurer les informations biométriques à partir du signal de réponse en tenant compte de l'impédance additionnelle de l'unité de charge (200), modifiée en fonction de la température mesurée par le capteur de température (390).

2. Appareil (10) de mesure d'informations biométriques selon la revendication 1, où :
la bioélectrode (130) comprend une électrode de travail et une électrode de référence, et l'unité de charge (200) est connectée en série à l'électrode de travail, ou est connectée en série à l'électrode de référence, ou est connectée en série respectivement à l'électrode de travail et à l'électrode de référence.

3. Appareil (10) de mesure d'informations biométriques selon la revendication 1 ou la revendication 2, où l'unité de charge (200) est disposée en série avec la bioélectrode (130) à l'intérieur d'un boîtier du module de mesure (300).

4. Appareil (10) de mesure d'informations biométriques selon l'une des revendications 1 à 3, où l'unité de charge (200) est formée sur le corps du capteur (100) et est formée comme traceur connectant électriquement la bioélectrode (130) et le module de mesure (300).

5. Appareil (10) de mesure d'informations biométriques selon l'une des revendications 1 à 4, où :
le capteur (100) et le module de mesure (300) sont raccordés de manière séparable l'un à l'autre, et
l'unité de charge (200) est formée en tant que borne de connexion (210, 230) connectant électriquement le capteur (100) et le module de mesure (300) lorsque le capteur (100) et le module de mesure (300) sont raccordés.

6. Appareil (10) de mesure d'informations biométriques selon l'une des revendications 1 à 5, où le module de mesure (300) comprend en outre un filtre passe bande de fréquence basse prévu pour filtrer une composante haute fréquence du bruit à partir du signal de réponse dont le niveau du bruit est réduit par l'unité de charge (200).

7. Appareil (10) de mesure d'informations biométriques selon l'une des revendications 1 à 6, où l'unité de charge (200) est une résistance de 10KΩ à 10GΩ.
